(19) Europäisches Patentamt
European Patent Office
Office européen des brevets

(11) Publication number: **0 002 963**
**B1**

(12) **EUROPEAN PATENT SPECIFICATION**

(45) Date of publication of patent specification: **21.12.83**

(51) Int. Cl.³: **G 01 N 33/50,**
G 01 N 21/64, C 07 G 7/00

(21) Application number: **78300913.7**

(22) Date of filing: **28.12.78**

(54) **Aqueous stabilized fluorescent labels, proteins labelled therewith and methods of use.**

(30) Priority: **28.12.77 US 865274**

(43) Date of publication of application:
**11.07.79 Bulletin 79/14**

(45) Publication of the grant of the patent:
**21.12.83 Bulletin 83/51**

(84) Designated Contracting States:
**DE FR GB**

(56) References cited:
**AU - D - 8 482 875**
**BE - A - 843 647**
**DE - A - 2 628 158**
**DE - A - 2 641 713**
**FR - A - 2 295 426**
**US - A - 3 853 987**
**US - A - 3 910 654**

**I. Phys. Chem. 68; 3324-3346 (1964)**

(73) Proprietor: **EASTMAN KODAK COMPANY**
**343 State Street**
**Rochester New York 14650 (US)**

(72) Inventor: **Frank, David Stanley**
**127 Harrington Drive**
**Rochester New York (US)**
Inventor: **Sundberg, Michael William**
**77 Highledge Drive**
**Penfield New York (US)**

(74) Representative: **Pepper, John Herbert et al,**
**KODAK LIMITED Patent Department P.O. Box 114**
**190 High Holborn**
**London WC1V 7EA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 002 963 B1**

# Aqueous stabilized fluorescent labels, proteins labelled therewith and methods of use

The present invention relates to fluorescent labels which are useful for labelling various proteinaceous species and more particularly fluorescent labels useful for the preparation of immunoreagents comprising fluorescently labelled antigens or antibodies.

Immunoassay is a field where sensitivity is of prime importance due to the low analyte levels that are measured. Radioimmunoassay sensitivity is limited to $10^{-12}$M and is more often only in the $10^{-8}$ to $10^{-10}$M range. In addition, radiolabels suffer from the drawbacks of short half life and handling hazards.

The sensitivity of fluorescence assays, although theoretically very high, is limited by the presence of background fluorescence. In many situations, it is impossible to reduce the background sufficiently (by appropriate filtration and other techniques known in the art) to obtain the desired sensitivity.

Time resolution offers an independent means of isolating the specific fluorescent signal of interest from nonspecific background fluorescence. This can be done if the label has much longer-lived fluorescence than the background, and if the system is illuminated by an intermittent light source such that the long-lived label can be measured during the dark period subsequent to the decay of the short-lived background. Such techniques are described in greater detail in German Offenlegungschrift 2,628,158.

The long-lived fluoresence (0.1—5 msec) of the aromatic diketone chelates of certain rare earth metals, for example, europiumbenzoylacetonate and europiumbenzoyltrifluoracetonate, has been known for some time. The chelating agent absorbs light and transfers it to the metal ion, which fluoresces. German OLS 2,628,158 describes the use of time resolution in fluorometric immunoassays (FIA) through the use of fluorescent labels whose emissions are long lived as compared to those of species which produce background interferences in such assays. This publication also provides a useful discussion of the techniques of FIA and its advantage over other immunoassay techniques such as radioimmunoassay (RIA).

The fluorescent immunoreagents described in German OLS 2,268,158 comprise at least one member of the immune-complement system, i.e., an antibody or an antigen, "conjugated" with a rare earth chelate. Such "conjugation" can be achieved in one of two ways:

(1) by first labeling, i.e., attaching the rare earth chelate to the antigen as described in "Fluorescent Antibody Techniques and Their Application" by A. Kawamura, Ed., University Park Press, Baltimore, Maryland, 1969, and then adding antibody to the conjugated antigen whereby the antibody and antigen join in the usual fashion, or:

(2) by covalent bonding of the antibody to the chelate via a chemical group which binds to both antibodies and the chelates.

The problem with immunoreagents of the type described in German OLS 2,628,158 is that the fluorescent labeling species, namely the rare earth chelates, are quenched, i.e., their fluoresence is extinguished, when contacted with water. This problem, hereinafter referred to as an "aqueous stability" problem, is particularly serious because a principal use for fluorescent labelled immunoreagents is in the assay of aqueous biological liquids such as blood and serum. If aqueous stability could be conferred on these materials, they would be useful as fluorescent labels for these biological liquids, thus allowing increased fluorescence immunoassay sensitivity by the use of time resolution of signal from background.

Australian Patent Application No. 84828/75 describes methods of making aqueous loaded polymer latices of which the dispersed phase comprises a polymer and a hydrophobic compound such as a colour-forming coupler, ultra-violet absorbing compound or anti-oxidant.

Belgian Patent Publication No. 843,647 describes scintillation counting compositions comprising polymeric particles derived from latices and loaded with at least one hydrophobic fluor, as well as methods for preparing such latices.

Leif, R. C. et al, Clinical Chemistry, Vol. 23, No. 8 (1977) suggest techniques for stabilizing rare earth chelates for use in aqueous liquids. The method generally comprises attaching a chelating agent to the surface of a polymeric bead and then chelating several other chelating agents and the bead-attached chelating agent to a single rare earth atom.

U.S. Patent No. 3,853,987 suggests the incorporation of "tracer" molecules throughout the volume of particles of acrylic acid derivatives in a latex and use of such particles as labels for immunoreagents. Although this patent teaches that the tracer is incorporated throughout the particle volume, there is no teaching of how the "tracer", fluorescent or radioactive, is incorporated into the particle. The only teachings are to attaching such "tracers" to acrylic polymers or adhering the "tracers" to the surface of such polymers.

It has now been discovered that the incorporation of fluorescent rare earth chelates into polymeric particles derived from latices of the type described, for example in the above-mentioned Belgian Patent 843,647 and Australian Application 84828/75 surprisingly eliminates the fluorescent quenching of these fluorescent rare earth chelates by aqueous liquids. Accordingly, the present invention provides a class of highly efficient, aqueous stabilized fluorescent labels for proteinaceous species, especially antigen and antibody immunological species. The present invention also provides a new class of

proteinaceous reagents, especially immunological reagents, bearing these highly useful fluorescent labels.

The present invention provides a particulate fluorescent label characterized by a fluorescent rare earth chelate incorporated into polymeric latex particles derived from a loadable latex wherein the rare earth chelate comprises up to 7.5% by weight of the particles, wherein the loadable latex has a polymeric discontinuous phase that consists essentially of polymer polymerized from one or more ethenic monomers and has an aqueous continuous phase and exhibits essentially no visible coagulation or settling out when 250 ml of the latex containing from 10 to 20 weight percent dispersed phase is added at a steady uniform rate with moderate stirring at 25°C to an equal volume of acetone over a 1 minute period and subsequently allowed to stand at 25°C for 10 minutes, whereby the label is stable in aqueous media, i.e. the fluorescence of the chelate is not quenched when the label is immersed in or otherwise exposed to an aqueous medium.

In another aspect, the present invention provides a fluorescently labelled protein comprising protein adsorbed or covalently bound to an aqueous stabilized fluorescent label as defined above.

In still another aspect, the present invention provides a fluorescently labelled immunoreagent comprising antigen or antibody adsorbed or covalently bound to an aqueous stabilized fluorescent label as defined above.

In yet another aspect, the present invention provides a method for performing an immunoassay using labelled immunoreagents, characterized by using as the labelled immunoreagent antigen or antibody adsorbed or covalently bound to an aqueous stabilized fluorescent label as defined above.

The present invention also provides a method of making an aqueous stabilized fluorescent label as described above comprising forming a solution of a fluorescent rare earth chelate in a water-miscible solvent and thereafter admixing a loadable polymeric latex, as defined above, whereby the fluorescent rare earth chelate is incorporated into the hydrophobic particles of the latex in amounts of up to 7.5% by weight of the particles.

Thus, the invention describes long-lived fluorescent rare earth chelates prepared by incorporating chelates of the rare earth metals, preferably europium and terbium, into latex particles. The chelating agent strongly absorbs light and efficiently transfers energy to the metal. The latex configuration confers aqueous stability to fluorescent rare earth chelates which in the past have been subject to quenching in aqueous liquids. The polymeric particles derived from the latex and having the rare earth chelate incorporated therein can then be used as fluorescent labels to form labelled reagents. This is accomplished by adsorbing or covalently binding antigens, antibodies, plant lectins, carbohydrates or other such proteinaceous compounds to the surface of the polymeric latex particles.

The use of these long-lived fluorescent labels makes it possible to take advantage of time resolution as a method of reducing background in, for example, fluorescence immunoassay systems. Furthermore, when the fluorescent rare earth chelate is incorporated into the latex particle according to the technique described in the aforementioned Belgian Patent No. 843,647, high levels of such chelate can be associated with antigen or antibody attached to the surface of the particle thereby producing an immunoreagent which demonstrates the reactivity of a single immunological unit, i.e., a single antigen or antibody, having a large quantity of label attached thereto, thus even further increasing the efficiency of the fluorometric immunoassay.

When used herein with reference to fluorescent rare earth chelates, the term "stabilized" means that the fluorescence of the chelate is not quenched when the label is immersed in or otherwise exposed to an aqueous medium.

Generally, any rare earth chelate which demonstrates fluorescent behavior is a candidate for use in the compositions described herein. A detailed discussion of such materials can be found in Chapter 8 of Sinha, Shiama P., Complexes of Rare Earths, Pergamon Press, 1966. The following references also provide an extensive discussion of rare earth chelates of the type useful in the compositions of the instant invention: Lytle, F. E., Applied Spectroscopy, *24*:319 (1970) and Filipescu, N., et al, J. Phys. Chem., *68*:3324 (1964).

The fluorescent compositions are comprised of chelates of the rare earth elements, preferably europium and terbium. Useful chelating agents for such rare earth elements have been discussed extensively in the literature referenced above and include by way of non-limiting examples:

1) 1-3-diketones, such as acetylacetonate, benzoylacetonate, benzoylbenzoate, trifluoro-2-furylacetylacetonate;

2) phthalates;

3) naphthalates; such as naphthoylmethide, naphthorylmethide;

4) dipyridines and terpyridines, such as 2,2′-bipyridine-1,1′-dioxide, 2,2′,6′,2″-terpyridine, 4,4′-dimethyl-2,2′-dipyridine;

5) phenanthrolines, such as phenanthroline isothiocyanate.

The 1,3-diketones are preferred for use herein because of their high transfer efficiency.

Specific chelates described by Filipsecu et al., supra, include europium dibenzoylmethide, europium p-methoxydibenzoylmethide, europium di-p-methoxydibenzoylmethide, europium p-nitrodibenzoylmethide, europium di-p-nitrodibenzoylmethide, europium di-m-methoxydibenzoylmethide, europium m-methoxydibenzoylmethide, europium m-nitrodibenzoylmethide,

europium di-m-nitrobenzoylmethide europium p-phenyldibenzoylmethide, europium di-p-fluorodibenzoylmethide, europium difuroylmethide, europium ditheonylmethide, europium di-1-naphthoylmethide, europium di-2-naphthoylmethide, europium diisonicotylmethide, europium benzoylacetonate, europium benzoyltrifluoroacetonate, europium theonyltrifluoroacetonate, europium acetylacetonate, europium trifluoroacetylacetonate, europium hexafluoroacetylacetonate, terbium dibenzoylmethide, terbium p-methoxydibenzoylmethide, terbium di-m-methoxydibenzoylmethide, terbium m-nitrodibenzoylmethide, terbium di-m-nitrodibenzoylmethide, terbium p-phenyldibenzoylmethide, terbium di-p-methoxydibenzoylmethide, terbium p-nitrodibenzoylmethide, terbium di-p-nitrodibenzoylmethide, terbium m-methoxydibenzoylmethide, terbium di-p-fluorodibenzoylmethide, terbium difuroylmethide, terbium ditheonylmethide, terbium di-1-naphthoylmethide, terbium di-2-naphthoylmethide, terbium diisonicotylmethide, terbium theonyltrifluoroacetonate, terbium benzoylacetonate, terbium benzoyltrifluoroacetonate, terbium acetylacetonate, terbium trifluoroacetylacetonate and terbium hexafluoroacetylacetonate.

To enhance the fluorescent yield of such chelates it may be desirable and it is preferred herein to include in the stabilized label particles small amounts of Lewis bases as described by Kleinerman, et al., J. Chem. Phys., *41*:4009 (1964) and Halverson et al., J. Chem. Phys., *41*:157 (1964). Trioctylphosphine oxide (TOPO) is a preferred Lewis base, although any of the other fluorescence enhancing materials described in the aforementioned Kleinerman and Halverson publications are similarly useful and may be preferred in certain environments of use.

The latices of the present invention are preferably, though not necessarily, prepared using the materials and methods described in the aforementioned Belgian Patent No. 843,647. The method described generally involves gradually increasing the hydrophilicity of a solution of a hydrophobe in a water-miscible solvent in the presence of uncoagulated, undissolved, loadable polymeric latex particles to a point at which substantially no hydrophobe remains dissolved in the water-miscible solvent phase. The increase in hydrophilicity is accomplished by adding water to the solution of hydrophobe in the water-miscible solvent. The advantage of incorporating the chelates using this technique is that concentrations of fluorescent rare earth chelate of up to about 7.5% by weight of the polymeric latex particle can be incorporated into the particle.

Further loading is achieved, and completed, by evaporation of the water-miscible solvent.

The preferred water-miscible solvents are organic and are those which:

(a) can be dissolved in (i.e., are "miscible" with) distilled water at 20°C to the extent of at least about 20 parts by volume of solvent in 80 parts by volume of water;

(b) have boiling points (at atmospheric pressure) above about 20°C;

(c) do not detrimentally react chemically with the loadable polymeric latices which are useful in the practice of this invention;

(d) do not dissolve more than about 5 weight percent of such loadable polymeric latices at 20°C; and

(e) act as solvents for the fluorescent rare earth chelates described hereinafter.

Examples of water-miscible solvents useful in the successful practice of the present invention include, solely by way of example, tetrahydrofuran, ethanol, methanol and acetone.

As used herein, the term "loadable polymeric latex" is defined as any polymeric latex which (i) has a polymeric discontinuous phase (particles), (ii) has an aqueous continuous phase, and (iii) does not coagulate or settle out when subjected to the following test:

At 25°C, slowly stir 250 ml of polymeric latex containing from about 10 to about 20 weight percent dispersed phase into an equal volume of acetone. The addition should take place over 1 minute at a steady, uniform rate, while the acetone is being stirred moderately. Discontinue the agitation and let the resulting blend stand at about 25°C for 10 minutes. At the end of that time observe the blend. A "loadable polymeric latex" is one which exhibits essentially no visible coagulation or settling out under these test conditions.

Although any polymeric latex which will meet conditions (i)—(iii) above is useful, polymeric latices which are particularly useful in the successful practice of the present invention are those polymeric loadable latices wherein the discontinuous phase (particles) comprises a polymer polymerized from

(a) from 0 to 100 weight percent of a styrene monomer having the formula

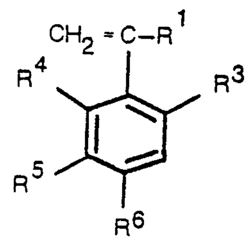

wherein $R^1$ is hydrogen or methyl; $R^3$ and $R^4$ are hydrogen or lower alkyl of 1 to 4 carbon atoms; $R^5$ and $R^6$ are hydrogen, substituted carbonyl or sulfonyl, e.g. haloalkylcarbonyl, vinyl sulfonyl, or lower alkyl of

1 to 4 carbon atoms which can be substituted in the alpha position, e.g., with halogen, alkyl sulfonyl halide, or $R^5$ with $R^4$ can constitute the atoms necessary to complete a fused benzene ring; of which particularly useful examples are styrene, vinyltoluene, 2-vinylmesitylene, 1-vinylnaphthalene, (2-chloroethylsulfonylmethyl)styrene, and meta- and para-chloromethylstyrene;

(b) from 0 to about 95 weight percent of units derived from one or more ethenic monomers of the formula

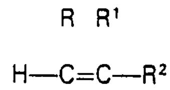

wherein R is hydrogen or alkyl containing 1 to about 5 carbon atoms; $R^1$ is hydrogen or methyl; and $R^2$ is hydrogen, halogen, methyl, cyano, the group

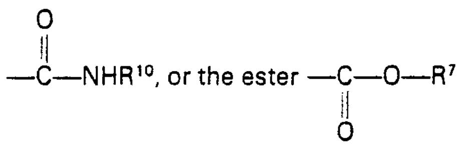

wherein $R^7$ is an aliphatic group containing from 1 to 6 carbon atoms which can be substituted with an amine group which can be further substituted to give a quaternary ammonium group; and $R^{10}$ is hydrogen or $R^7$; and

(c) from about 0 to about 10 weight percent of a hydrophilic ethenic monomer containing a sulphonic acid group, or an ammonium or alkali metal salt thereof; said ethenic monomer preferably having a molecular weight of, at most, about 300 provided that at least one of (a), (b) and (c) is present.

In addition, other monomers of the class (a), (b) or (c), can be added, where copolymerization is possible.

It should be noted that the ratios of monomers set out herein are based upon the relative proportions of various monomers as they are charged into the polymerization reactor in a conventional free radical polymerization process. Products from such reactions may vary to some extent in the ratios derived from the charged monomers for various reasons which are well known to those skilled in the art of manufacturing synthetic polymeric latices. While "loadable" polymeric latices can be made from one, two, three, four, or even more different monomers, those which are preferred for use in the practice of this invention are generally comprised of two to four different monomers, depending upon the particular properties desired in the final products of the invention. As the manufacture of latices of this type is well known, details of such procedures need not be described herein, except to point out that the preferred loadable polymeric latices described above are generally prepared via free radical initiated reactions of monomers dispersed in an aqueous medium with one or more appropriate surfactants. See, for example, U.S. Patents 2,914,499; 3,033,833; 3,547,899 and Canadian Patent 704,778.

The generic formula for a preferred subclass of hydrophilic ethenic monomers containing the sulphonic acid group is

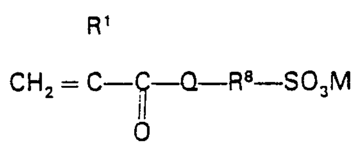

where $R^1$ is methyl or hydrogen; $R^8$ is a straight or branched-chain alkylene group of 1 to 5 carbon atoms, e.g. methylene, ethylene, 2-methylethylene, trimethylene, tetramethylene, 2,2-dimethylethylene, 3-methyl propylene, 3,3-dimethyl propylene, 2-methyl propylene and the like; M is ammonium, hydrogen, or alkali metal cation; and Q is O or NH.

Polymers which are still further preferred for use as loadable polymeric latices are those wherein (i) the ethenic monomer of the formula

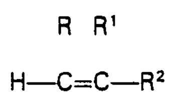

is acrylonitrile or an ester selected from the group consisting of methyl, ethyl, propyl and n-butyl acrylates and methacrylates, and (ii) the hydrophilic ethenic monomer is selected from those having a sulphonic acid group (or water soluble salt thereof) preferably attached to a terminal carbon atom such as, for example, those having the following structure:

(1) 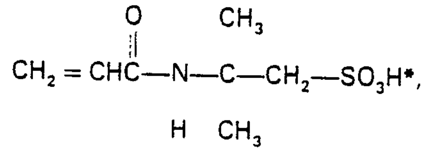

(2) 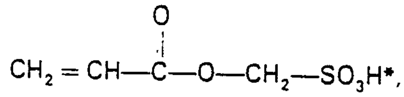

(3) 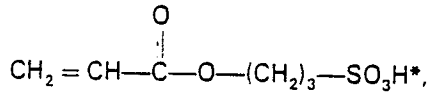

(4) 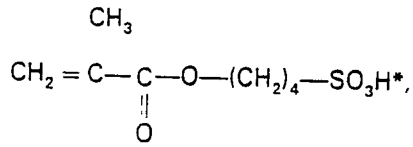

(5) 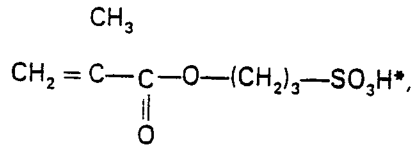

(6) 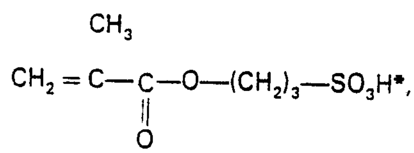

(7) 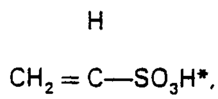

(8) 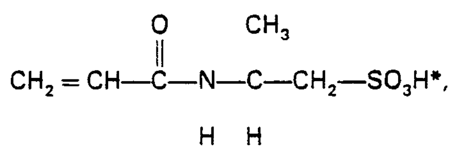

(9) 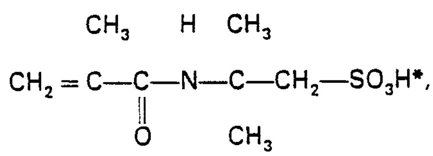

(10) 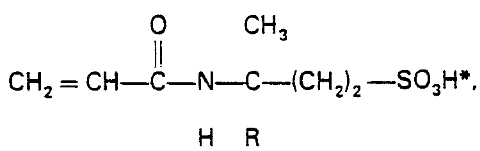 where R is H or $CH_3$.

(* = in place of H can be an alkali metal cation, preferably $Na^+$ or $K^+$, or ammonium ion.)

Highly preferred loadable polymers include latices comprising:

1) Poly(n-butyl acrylate-co-styrene-co-2-acrylamido-2-methylpropanesulphonic acid) (30:65:5)

2) Poly(n-butyl acrylate-co-2-acrylamido-2-methylpropanesulphonic acid) (90:10)

3) Poly[styrene-co-(2-chloroethylsulphonylmethyl)styrene-co-2-acrylamido-2-methylpropanesulphonic acid] (88:7:5)

4) Poly[styrene-co-acrylamide-co-(2-chloroethylsulphonylmethyl)styrene-co-2-acrylamido-2-methylpropanesulphonic acid] (75:14:6:5)

5) Poly(n-butyl acrylate-co-styrene-co-m+p-chloromethylstyrene-co-2-acrylamido-2-methylpropanesulphonic acid) (33:30:35:2); and

6) Poly[n-butyl acrylate-co-styrene-co-m+p-chloromethylstyrene-co-2-(methacryloyloxy)ethyltrimethylammonium methosulphate] (35:50:10:5)

7) Poly[styrene-co-acrylamide-co-(2-chloroethylsulphonylmethyl)styrene] (70:25:5)

From the foregoing description it is evident that many combinations of monomers can be used in the manufacture of synthetic polymeric latices in accordance with the most preferred embodiments of this invention. It must be pointed out, however, that many polymeric latices are not "loadable polymeric latices" as set out above. For this reason, it is recommended that, before a given latex is assumed to be "loadable", the latex be tested by the test procedure set out above. The use of this test is also recommended as a control procedure because of the relatively low level of batch-to-batch reproducibility that sometimes occurs in the commercial manufacture of polymeric latices. A preferred method for manufacturing loadable polymeric latices is described below, preceding the Examples.

The dispersed polymeric particles comprising the discontinuous phase of the latex, formed in the manner described above, have an average diameter of from about 0.01 to about 0.2 $\mu$m. Thus, the latex can be considered a colloidal dispersion. Furthermore, a preferred concentration range of latex particles is such that the polymer portion thereof, calculated apart from the fluorescent rare earth chelate, gives a concentration of from 0.03 g/cc to 0.25 g/cc.

The concentration of fluorescent rare earth chelate in the latex particle will also vary to some extent depending upon the particular utility for which the particle is intended. Thus, an individual particle may contain at a minimum a single fluorescent rare earth chelate molecule and as a maximum about 7.5% by weight of the chelate. The use of chelate concentration levels at the high end of this range makes possible the detection of analyte at levels as low as $10^{-14}$ — $10^{-15}$ M. A molar ratio of 1 metal:3 chelating agent:2 Lewis base is a preferred embodiment. The latex particle size can be chosen based on the specific purpose for which the fluorescent label is intended; however, 0.01 to 0.15 $\mu$m diameter latex particles are preferred for most immunoassays.

## Preferred Process of Manufacture of the Latex

With respect to the preferred process of manufacturing the compositions of the present invention, the order of addition of the loadable polymeric latex into the solution of fluorescent rare earth chelate dissolved in water-miscible solvent is important. Reversing the order results in the coagulation and settling out of the latex or the accumulation of a large proportion of the fluorescent rare earth chelate outside the latex particles in a much less desirable or less useful form.

In the manufacture of the loaded polymeric latex compositions employed in the practice of this invention, generally the relative volume of

(a) loadable polymeric latex; and

(b) solution of fluorescent rare earth chelate(s) (in water-miscible solvent) which are intermixed in the required manner, are not believed critical. Thus, so long as some loadable polymeric latex particles are present in the solution during that interval of time in which the fluorescent rare earth chelate is forced out of solution (because of the increasing hydrophilicity of the solution, as described above), some loaded polymeric latex particles will be created. For example, one embodiment of the present generic process involves, stepwise,

(a) the introduction of a quantity of loadable polymeric latex which is not sufficient to affect the hydrophilicity of the solution of fluorescent rare earth chelate to the extent necessary to force the chelate out of solution; and

(b) adding enough water to the resulting mixture to effect the desired transfer of fluorescent rare earth chelate from the water-miscible solvent into the latex particles.

In this way, loaded polymeric latices containing relatively larger proportions of fluorescent rare earth chelate per particle can be made using a relatively dilute solution of chelate. Thus, it can be seen that there is more than one technique whereby the necessary increase in the hydrophilicity of the solution of fluorescent rare earth chelate (during the period in which it becomes insoluble in such solution) can be obtained. For this reason, when the phrase "at least sufficient water to cause the fluorescent rare earth chelate to become insoluble in the solution" (with reference to the essential step of this process) is used herein, the "water" referred to in that phrase means not only water alone, but also the "aqueous" portion of a loadable polymeric latex as described hereinbefore, as well as water in the form of a solution of one or more dissolved salts.

However, it is generally preferred that when the dispersed phase polymer "solids" (i.e. the particles) of the loadable polymeric latex is above 10 weight percent, the relative amount of fluorescent rare earth chelate solution that is blended with such latex should be between about 50 and about 200 parts by volume per 100 parts by volume of loadable polymeric latex; and still more preferably, about one part by volume of fluorescent rare earth chelate solution per part by volume of loadable polymeric latex, particularly when the latex contains from 12 to 20 weight percent of polymeric latex particles. The actual optimum amount of time necessary to carry out the gradual mixing of (i) the latex and (ii) the fluorescent rare earth chelate solution in accordance with the present process will vary in any given instance, depending upon such factors as

(a) the identity of the loadable polymeric latex, fluorescent rare earth chelate, and water-miscible solvent;

(b) the relative concentrations of fluorescent rare earth chelate and polymeric latex particles in the respective materials to be mixed, as well as;

(c) the relative amounts of loadable polymeric latex and fluorescent rare earth chelate solution. However, it is generally preferred that the gradual intermixing of loadable polymeric latex into the fluorescent rare earth chelate solution take place over at least about 10 seconds, particularly in those instances in which the polymeric "solids" content of the loadable polymeric latex is above about 12 weight percent. Too fast intermixing has been found to result in formation of a second solid phase in the system and/or coagulation or settling of the polymeric latex particles. Gradual intermixing over at least about 20 seconds is still further preferred.

Generally, after a useful loaded polymeric latex composition, as described hereinbefore, has been formed initially, some or all of the water-miscible solvent can, optionally, be removed from the composition without harming the valuable utility of such loaded polymeric latex composition. Removal of water-miscible solvent can preferably be accomplished by evaporation under way of a wide variety of conditions (at temperatures below about 40°C, for example), preferably under reduced pressure. Preferably, at least about half of the water-miscible solvent is removed from the initial compatible blend (of loadable polymeric latex/fluorescent rare earth chelate/water-miscible solvent) to thereby form one of the preferred useful loaded polymeric latices. Such preferred latices retain their "latex" characteristics; that is, they have an aqueous continuous phase which, optionally, contains some of the water-miscible solvent (but, preferably, not more water-miscible solvent than about 30 weight percent of said continuous phase), and a dispersed phase comprising loaded polymeric latex particles in which the fluorescent rare earth chelate is uniformly distributed. Removal of the water-miscible solvent and/or some water from the initial blend of latex plus water-miscible solvent, of course, results in a composition having a higher solids content.

When it is desired to improve the stability of a loaded polymeric latex to inhibit the tendency of the latex to settle out gradually upon prolonged storage, it can be intermixed with an aqueous solution of a hydrophilic colloid such as gelatin. Such an embodiment is specifically preferred. A preferred minimum amount of hydrophilic colloid and/or a starch such as grafted starch in the resulting mixtures is about 1 weight percent based on the weight of the loaded polymeric latex, although more hydrophilic colloid can be used, if desired, to form a stabilized latex product.

The following is an illustrative, non-limiting example of the preparation of poly(n-butyl methacrylate-co-styrene-co-2-acrylamid-2-methylpropane sulfonic acid) in a charge weight ratio of 50:40:10, which provides suitable "loadable" polymeric latex particles as described above:

To a one-litre addition flask were added 200 g of n-butyl methacrylate, 160 g of styrene, and a solution consisting of 7.7 g of sodium hydroxide, 350 ml water, 40 g of 2-acrylamido-2-methylpropane sulfonic acid, and 2 g of a 40% solution of "Triton 770", the registered Trade Mark of an anionic sodium salt surfactant of an alkylaryl polyether sulfate in liquid form manufactured by Rohm and Haas. The mixture was stirred for 30 min. prior to the addition process. In a 250 ml addition funnel was added 200 ml $H_2O$ containing 2 g of $K_2S_2O_8$. Both the addition flask and the addition funnel were connected to a 3 liter reaction flask containing 800 ml $H_2O$ and 4 g of "Triton 770" (40%) maintained at 95°C with stirring. To start the polymerization, 1.2 g of $Na_2S_2O_5$ was added to the reaction flask immediately followed by the addition of monomer mixture and $K_2S_2O_8$ solution. The period of addition was about 30 min. The polymerization was allowed to proceed for an additional 30 min. The latex was then cooled and dialyzed overnight to give a solids content of 13.8%.

Loading of the loadable polymeric latex with fluorescent rare earth chelate is accomplished as described hereinabove. The loaded polymeric latex particles can then be used to label a variety of protein-aceous species by binding the protein to the surface of the particle either by adsorption or by covalent bonding. Among the proteinaceous species which can be labelled in this fashion are enzymes, antigens, antibodies, plant lectins and similar compositions.

Techniques for binding the proteins to the surface of polymeric particles are well known in the art and an extensive quantity of patent and technical literature is directed to such techniques and specific linking groups for performing the attachment. German OLS 2,548,427 describes useful such techniques. Examples 1 and 2 below demonstrate useful adsorption and convalent bonding techniques according to the practice of the present invention.

When covalent bonding of the proteinaceous species to the polymer particle is desired, it is preferred to use in the manufacture of the polymeric latex a monomer which after particle formation retains a chlorobenzyl, chloroacetyl, chloroethylcarbonyl, chloroethylsulphonyl, acryloyl, or vinylsulphonyl group which can react with amino, amido, or sulphonamido groups on the enzyme, antibody, antigen, proteinaceous species or carbohydrates to be bound.

Accordingly, following attachment of the preotinaceous species, highly preferred fluorescent labelled polymers for use as the polymeric latex are those having the following structural formula,

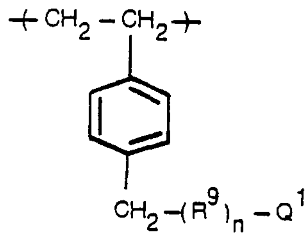

wherein $Q^1$ is the proteinaceous species, n is 0 or 1, and $R^9$ is a linking group selected from the group consisting of

(a) $—SO_2CH_2CH_2—$

(b) 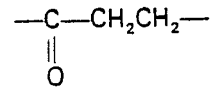

Other representative techniques for accomplishing binding are described in the following patents: U.S. 3,088,875; 3,766,013; 3,619,371; 3,809,613; 3,853,987; 3,963,441; 3,551,555; and 3,646,346. All of the aforementioned patents describe techniques for absorbing or covalently bonding proteinaceous species, especially antigens and antibodies, to a variety of polymeric species of the type which are useful in the polymeric latex particles employed in the practice of the present invention.

Once prepared as described hereinabove, the fluorescent latex particle labelled immunologically reactive species can be used in fluorescent immunoassays, particularly those which utilize temporal resolution of the specific detecting signal to distinguish from background as described in aforementioned German OLS 2,628,158. In this time-resolved mode (i.e. temporal resolution), the sample is excited in an intermittent fashion and information is accepted only during the dark cycle when the long-lived fluorescent label is still emitting strongly but when other sources of fluorescence have decayed. Discontinuous excitation can be achieved in a variety of ways, including pulsed laser, mechanical chopping of a continuous excitation beam, moving the sample in and out of the excitation beam, etc. Moreover, discontinuous excitation has the advantage of allowing the use of high radiant power without the adsorption of the large amount of energy by the sample, thus diminishing the probability of sample photodegradation.

Typical such fluorescent radioimmunoassay techniques wherein the immunoreagents described herein find utility are descirbed in U.S. Patent Nos. 4,020,151; 3,939,350; and 3,901,654.

The following non-limiting examples will serve to better illustrate the successful practice of the instant invention:

In all of the examples, the europium complex that is incorporated into the latex is europium (III) (theonyltrifluoroacetone)$_3$ along with trioctylphosphine oxide (TOPO) in the ratio of 1 Eu complex: 2 TOPO.

## EXAMPLE 1

### The Adsorption of Bovine Gamma Globulin to Loaded Polymeric Latex Particles

Bovine gamma globulin (BGG) (50 mg) is dissolved in 100 ml of distilled water. To it is added 250 $\mu$l of 7% suspension of europium-incorporated latex, poly(n-butyl acrylate-co-styrene-co-2-acrylamide-2-methylpropanesulphonic acid) (30:65:5), and the pH adjusted to 9.0 using a carbonate buffer. The mixture is chromatographed through BioGel A-5 (available from Bio Rad, Richmond, California) resin (50 ml, 100—200 mesh) and eluted with water. The latex is filtered through a 0.22 $\mu$m Millipore filter before use, and can be specifically precipitated with an antibody that reacts with BGG. 'Millipore" is a Trade Mark.

## EXAMPLE 2

### Covalent Attachment of Antigen to Loaded Polymeric Latex Particles

A. *Binding of BGG to Europium-Incorporated Latex*

BGG (100 mg) is dissolved in 200 ml of distilled water with stirring. Next, the pH is adjusted to 9.6 with 6 N sodium hydroxide, and the solution is cooled to 11°C in a water bath shaker. 0.5 ml of a 7% suspension of europium-incorporated latex, poly[styrene-co-(2-chloroethylsulphonylmethyl) styrene-co-2-acrylamido-2-methylpropanesulphonic acid] (88:7:5), is added, and the reaction mixture is shaken for 72 hours at 11°C. To this is added 0.1 ml ethanolamine, and the mixture is shaken an additional 24 hours at 11°C. The mixture is dialyzed against running distilled water for 72 hours. After dialysis, the dialyzate is chromatographed two times through Bio-Gel A-5 resin (50 ml, 100—200 mesh) and eluted with water each time. A yield of 87.5 mg is obtained after lyophilization.

B. *Binding of L-Thyroxine to Europium-Incorporated Latex*

L-thyroxine (0.15 g) is dissolved in 60 ml of stirred distilled water at pH 12 (adjusted with 6 N sodium hydroxide). Nine ml of a 7% suspension of 0.05 $\mu m$ diameter europium-incorporated latex, poly[styrene-co-(2-chloroethylsulphonylmethyl)styrene-co-2-acrylamido-2-methylpropanesulonic acid] (88:7:5), is added, and the pH of the reaction mixture readjusted to 12 with 6 N sodium hydroxide. The reaction mixture is shaken for 4 days at 11°C. Butylamine (0.5 ml) is added and shaking is continued for 24 hours. The reaction mixture is dialyzed against running distilled water for 3 days and then against 4 liters of 1% aqueous bovine serum albumin for an additional 3 days. The dialyzed reaction mixture is slowly passed twice through 50 ml of Bio-Gel A-5 resin. The eluate is passed through a sintered glass funnel (coarse grade) then dialyzed against running distilled water for 4 days and freeze-dried. The product weighs 0.5 g. Iodine calc. 20.5% (complete reaction of polymer); found 3.8%.

C. *Binding of L-Thyroxine-Rabbit Gamma Globulin to Europium-Incorporated Latex*

*Part 1.* Preparation of L-thyroxine-rabbit gamma globulin: In 200 ml of stirred distilled water 0.5 g ($3.3 \times 10^{-6}$ mole) rabbit gamma globulin (RGG) is dissolved. 0.3 g ($7.1 \times 10^{-4}$ mole) of 1-cyclohexyl-3-(2-morpholinoethyl)-carbodiimide metho-p-toluenesulfonate is added to the stirred solution.

0.4 g ($5.2 \times 10^{-4}$ mole) of L-thyroxine ($T_4$) is dissolved in 120 ml of N,N-dimethylformamide. The pH of the solution is adjusted to 6.5 (with dilute hydrochloric acid), and this solution is added slowly to the stirred protein solution over a period of 20 min. Upon completion of the addition, an additional 0.4 g of the carbodiimide is added, and the reaction mixture is stirred overnight at room temperature. The reaction mixture is dialyzed against running distilled water for 4 days, then against 4 litres of 1% aqueous bovine serum albumin for 3 days. The dialyzate is freeze-dried. It weighs 0.53 g. Spectrophotometric analysis for L-thyroxine content indicated a ratio of 14 L-thyroxine units per rabbit gamma globulin. Iodine calculated on 14 units, 4.4%; found, 4.7%.

*Part 2.* Binding of L-thyroxine-rabbit gamma globulin to europium-incorporated latex: L-thyroxine-rabbit gamma globulin (0.1 g) prepared as described above was dissolved in 200 ml of distilled water. The pH is adjusted to 9.5 with 6 N sodium hydroxide and 0.5 ml of a 7% suspension europium-incorporated latex, poly [styrene-co-(2-chloroethylsulphonylmethyl)styrene-co-2-acrylamido-2-methylpropanesulphonic acid] (88:7:5), is added. The reaction mixture is shaken for 3 days at 11°C. Ethanolamine (0.1 ml) is added, and the shaking is continued for 24 hours. The reaction mixture is dialyzed against running distilled water for 48 hours then chromatographed twice on 50 ml of Bio-Gel A-5 resin. The final volume of liquid is approximately 200 ml (0.2 mg/dl solids). Iodine percentage calculated for 78 $T_4$-RGG/latex, 1.4%; found 0.88%. $T_4$-RGG/latex = 40.

## EXAMPLE 3

### Specific Binding of the Fluorescent Latex Labelled Antigen to Antibody

A. *Agglutination of the Latex-Bound Antigen by Antiserum*

Ten microliters of a 0.02% suspension of 0.1 $\mu m$ diameter europium-incorporated latex particles to which bovine gamma globulin was covalently bound were incubated in snap cap plastic tubes with 25 $\mu l$ of rabbit antiserum to bovine gamma globulin in a total volume of 145 $\mu l$ (phosphate-buffered saline, pH 7.5). After several hours, a precipitate was observed which was fluorescent when illuminated with light at 365 nm. In a parallel test with nonimmune rabbit serum, there was no precipitate.

B. *Specific Binding of Antigen-coated Fluorescent Latex Particles to Antibody-Coated Micrometer Size Beads*

Test solutions are prepared in 1.5 ml snap cap plastic tubes containing the following:

1. 100 $\mu l$ 3% Triton X-100 (Rohm and Haas) in 0.075 M sodium barbital buffer, pH 8.5, and
2. 100 $\mu l$ of a 0.1% suspension (0.075 M sodium barbital buffer, pH 8.5) of 0.1 $\mu m$ diameter europium-incorporated latex particles, poly[stryene-co-(2-chloroethylsulphonylmethyl)styrene-co-2-acrylamido-2-methylpropanesulphonic acid] (88:7:5), to which bovine gamma globulin had been covalently bound (i.e. antigen-coated latex particles and
3. 10, 5, 2.5, 1.25, 0.63, or 0.31 mg beads (6 $\mu m$ diameter) to which the gamma globulin fraction of (rabbit) anti-BGG had been adsorbed (i.e., antibody-coated beads), or
4. 10, 5, 2.5, 1.25, 0.63 or 0.31 mg beads to which nonimmune rabbit gamma globulin had been adsorbed (i.e. control beads).

The tubes are allowed to stand for one hour after addition of the control or antibody-coated beads. The 6 $\mu m$ beads settle, and the tubes are observed by UV (365 nm) light for the distribution of latex. In all the control preparations, no red fluorescence due to europium can be observed in the settled beads. A slight film of latex is noticed at the solution-bead interface. In the preparations containing antibody-coated beads, significant fluorescence is noted in the settled bead volume, and diminution of the supernatant fluorescence is apparent when these tubes are compared to those in which comparable amounts of inactive beads were present.

C. *Response of the BGG-Coated Fluorescent Latex/Anti-BGG-Coated Bead System to BGG*

Into 1.5 ml snap cap plastic tubes are placed 100 $\mu l$ of a 10% suspension of the antibody-coated

latex described in Part B and 100 $\mu l$ of the 0.1% antigen-coated latex described in Part B and 100 $\mu l$ of barbital buffer containing bovine gamma globulin at $10^{-4}$, $5 \times 10^{-5}$, $2.5 \times 10^{-5}$, $1.25 \times 10^{-5}$, $6.25 \times 10^{-6}$, $3.13 \times 10^{-6}$, and 0 M. After incubating at room temperature for one hour, the beads settle and the europium fluorescence in the beads and supernatant can be measured when illuminated by 365 nm light. A regular monotonic increase in supernatant fluorescence and a concomitant diminution of bead fluorescence are noted with increasing concentrations of bovine gamma globulin. The midpoint in this series is between 1.25 and $2.5 \times 10^{-5}$ M bovine gamma globulin (which, when diluted 1:3 in the tube, gives a value of 4.1 to $8.3 \times 10^{-6}$ M).

Comparative Examples

The following example describes an attempt to load a polysaccharide bead with a europium chelate.

Materials: AH-Sepharose-4B, a bead-formed agarose gel having a wet diameter of 40—190 $\mu$ sold by the Pharmacia Fine Chemical Co. (Piscataway, New Jersey).

Two grams of a 10% suspension of AH-Sepharose-4B beads (in saline) were added to two grams of a 0.5% $Eu^{+3}$ solution in acetone with mixing at 25°C. The solvent was removed at 60°C. No filtration was attempted because the beads would have been removed in this process. A visible settling out occurred within 10 minutes. The beads were then subjected to ultraviolet radiation to detect the presence of fluorescene. None was visible. It was concluded that these beads are not loadable by the method of the present invention.

It has also been observed that through proteins such as antigens may be attached to these surfaces, it was not possible to differentiate precipitation that may have occurred as a result of immunological reaction (i.e. Antibody +Ag → Antibody Ag) from precipitation or settling out of the beads simply because they are heavy and not dispersible in solution.

It was concluded that these beads do not meet the requirements for suitable materials for the subject invention.

**Claims**

1. A particulate fluorescent label characterized by a fluorescent rare earth chelate incorporated into polymeric latex particles derived from a loadable latex wherein the rare earth chelate comprises up to 7.5% by weight of the particles, wherein the loadable latex has a polymeric discontinuous phase that consists essentially of polymer polymerized fron one or more ethenic monomers and has an aqueous continuous phase and exhibits essentially no visible coagulation or settling out when 250 ml of the latex containing from 10 to 20 weight percent dispersed phase is added at a steady uniform rate with moderate stirring at 25°C to an equal volume of acetone over a 1 minute period and subsequently allowed to stand at 25°C for 10 minutes, whereby the label is stable in aqueous media, i.e. the fluorescence of the chelate is not quenched when the label is immersed in or otherwise exposed to an aqueous medium.

2. The label as claimed in Claim 1 in which the fluorescent rare earth chelate is a chelate of europium or terbium.

3. The label as claimed in Claims 1 or 2 in which the polymeric latex particle comprises a (1) poly-(n - butyl acrylate - co - styrene - co - 2 - acrylamido - 2 - methylpropenesulphonic acid), (2) poly(n - butyl acrylate - co - 2 - acrylamido - 2 - methylpropanesulphonic acid) (3) poly[styrene - co - (2 - chloroethylsulphonylmethyl) - stryene - co - 2 - acrylamido - 2 - methylpropanesulphonic acid], (4) poly[styrene - co - acrylamide - co - (2 - chloroethylsulphonymethyl) - styrene - co - 2 - acrylamido - 2 - methyl - propanesulphonic acid], (5) poly(n - butyl acrylate - co - styrene - co - m + p - chloromethyl - styrene - co - 2 - acrylamido - 2 - methylpropanesulphonic acid), (6) poly[n - butyl acrylate - co - styrene - co - m + p - chloromethyl - styrene - co - 2 - (methacryloyloxy)ethyltrimethylammonium methosulphate], or (7) poly[styrene - co - acrylamide - co - (2-chloroethyl - sulphonylmethyl)styrene].

4. The label as claimed in any of the Claims 1 to 3 in which the fluorescent rare earth chelate is formed from a rare earth and a 1,3-diketone, phthalate, naphthalate, di-pyridine, terpyridine, p-benzoylbenzoate, p-benzoylacetonate, or phenanthraline chelating agent.

5. A fluorescently labelled protein comprising protein absorbed or convalently bound to an aqueous stabilized fluorescent label as claimed in any of the Claims 1 to 4.

6. The labelled protein as claimed in Claim 5 in which the protein is an antibody, antigen, or enzyme.

7. A fluorescently labelled immunoreagent comprising an antigen or antibody adsorbed or covalently bound to an aqueous stabilized fluorescent label as claimed in Claims 1 to 4.

8. A method of making an aqueous stabilized fluorescent label as claimed in Claims 1 to 4 comprising forming a solution of a fluorescent rare earth chelate in a water-miscible solvent and thereafter admixing a loadable polymeric latex, as defined in Claim 1, whereby the fluorescent rare earth chelate is incorporated into the hydrophobic particles of the latex in amounts of up to 7.5% by weight of the particles.

9. A method of making a labelled immunoreagent as claimed in Claim 7 wherein an aqueous stabilized fluorescent label as claimed in any of the Claims 1 to 4 is admixed with an aqueous solution of an antigen or antibody.

10. A method of performing an immunoassay using a labelled immunoreagent as claimed in Claim 7 wherein after admixture with a test sample the fluorescence of the supernatant liquid or any precipitate is measured.

11. The method as claimed in Claim 10 wherein the fluorescence is measured using a temporal resolution technique.

12. A method for performing an immunoassay using fluorescently labelled immunoreagents, characterised in that, the labelled immunoreagent is an antigen or antibody adsobed or covalently bound to an aqueous stabilized particlate fluorescent label as claimed in Claim 1.

**Patentansprüche**

1. Teilchenförmiges fluoreszierendes Markierungsmittel, gekennzeichnet, durch ein in Polymer-Latexteilchen eines beladbaren Latex eingebautes fluoreszierendes Seltenes-Erd-Chelat, bei dem das Seltene-Erd-Chelat bis zu 7,5 Gew.-% der Teilchen ausmacht und bei dem das beladbare Latex eine polymere diskontinuierliche Phase besitzt, die im wesentlichen aus einem durch Polymerisation eines oder mehrerer äthenischer Monomere erhaltenen Polymeren besteht, sowie eine wässrige kontinuierliche Phase, und im wesentlichen keine sichtbare Koagulation oder keinen Niederschlag erkennen läßt, wenn 250 ml des 10 bis 20 Gew.-% der dispergierten Phase enthaltenden Latex bei einer konstanten, gleichmäßigen Geschwindigkeit unter mäßigem Rühren bei 25°C dem gleichen Volumen an Aceton innerhalb einer Minute zugesetzt und dann 10 Minuten lang bei 25°C stehengelassen werden, wodurch das Markierungsmittel in wässrigen Medien stabil ist, d.h. die Fluoreszenz des Chelats nicht gelöscht wird, wenn das Markierungsmittel in ein wässriges Medium eingetaucht oder diesem in anderer Weise ausgesetzt wird.

2. Markierungsmittel nach Anspruch 1, in dem das fluoreszierende Seltene-Erd-Chelat ein Chelat des Europiums oder Terbiums ist.

3. Markierungsmittel nach Ansprüchen 1 oder 2, in dem das Polymer-Latexteilchen enthält oder besteht aus einer (1) Poly(n - butylacrylat - co - styrol - co - 2 - acrylamido - 2 - methylpropansulphonsäure), (2) Poly(n - butylacrylat - co - 2 - acrylamido - 2 - methylpropansulphonsäure), (3) Poly[styrol - co - (2 - chloräthylsulphonylmethyl)styrol - co - 2 - acrylamido - 2 - methylpropansulphonsäure], (4) Poly[styrol - co - acrylamido - co - (2 - chloräthylsulphonylmethyl)styrol - co - 2-acrylamido - 2 - methylpropansulphonsäure], (5) Poly(n - butylacrylat - co - styyrol - co - m - p - chlormethylstyrol - co - 2 - acrylamido - 2 - methylpropansulphonsäure), (6) Poly[n - butylacrylat - costyrol - co - m+p - chlormethyl - styrol - co - 2 - (methacryloyloxy)äthyltrimethylammoniummethosulphat] oder (7) Poly[styrol - co - acrylamido - co - (2 - chloräthylsulphonylmethyl)styrol].

4. Markierungsmittel nach einem der Ansprüche 1 bis 3, in dem das fluoreszierende Seltene-Erd-Chelat aus einer Seltenen Erde und einem Chelatbildner aus einem 1,3-Diketon, Phthalat, Naphthalat, Di-pyridin, Terpyridin, p-Benzoylbenzoat, p-Benzoylacetonat oder Phenanthralin gebildet ist.

5. Fluoreszierend markiertes Protein aus einem Protein, das an ein wässriges stabilisiertes, fluoreszierendes Markierungsmittel gemäß einem der Ansprüche 1 bis 4 adsorbiert oder kovalent gebunden ist.

6. Markiertes Protein gemäß Anspruch 5, in dem das Protein ein Antikörper, Antigen oder Enzym ist.

7. Fluoreszierend markiertes Immunoreagens mit oder aus einem Antigen oder Antikörper, das bzw. der an ein wässriges stabilisiertes, fluoreszierendes Markierungsmittel gemäß einem der Ansprüche 1 bis 4 adsorbiert oder kovalent gebunden ist.

8. Verfahren zur Herstellung eines wässrigen, stabilisierten fluoreszierenden Markierungsmittels gemäß Ansprüchen 1 bis 4, bei dem man eine Lösung eines fluoreszierenden Seltenen-Erd-Chelats in einem mit Wasser mikschbaren Lösungsmittel erzeugt und daraufhin mit einem beladbaren Polymer-Latex gemäß Anspruch 1 vermischt, wodurch das fluoreszierende Seltene-Erd-Chelat in Gewichtsanteilen von bis zu 7,5 Gew.-% der Teilchen in die hydrophoben Teilchen des Latex eingebaut wird.

9. Verfahren zur Herstellung eines markierten Immunoreagens gemäß Anspruch 7, bei dem ein wässriges stabilisiertes, fluoreszierendes Markierungsmittel gemäß einem der Ansprüche 1 bis 4 mit einer wässrigen Lösung des Antigens oder Antikörpers vermischt wird.

10. Verfahren zur Durchführung einer Immunobestimmung unter Verwendung eines markierten Immunoreagens gemäß Anspruch 7, bei dem nach Vermischen mit der Testprobe die Fluoreszenz der überstehenden Flüssigkeit oder irgendeines Niederschlages gemessen wird.

11. Verfahren nach Anspruch 10, bei dem die Fluoreszenz unter Anwendung einer zeitlichen Auflösungstechnik gemessen wird.

12. Verfahren zur Durchführung einer Immunobestimmung unter Verwendung von fluoreszierend markierten Immunoreagentien, dadurch gekennzeichnet, daß das markierte Immunoreagens ein

Antigen oder Antikörper ist, der an ein wässriges stabilisiertes, teilchenförmiges, fluoreszierendes Markierungsmittel nach Anspruch 1 adsorbiert oder kovalent gebunden ist.

**Revendications**

1. Produit de marquage fluorescent, sous forme de particules, caractérisé par un chélate d'un métal de terre rare fluorescent incorporé dans des particules de latex de polymère, dérivées d'un latex pouvant être chargé, dans lesquelles le chélate de métal de terre rare représente jusqu'à 7,5% en masse des particules et dans lesquelles le latex pouvant être chargé présente une phase polymère discontinue constituée essentiellement d'un polymère discontinue constituée essentiellement d'un polymère discontinue constituée essentiellement d'un polymère polymérisé à partir d'un ou plusieurs monomères éthyléniques et une phase aqueuse continue, et ne présente pratiquement pas de coagulation ou de séparation visible lorsqu'on ajoute 250 ml du latex contenant 10% à 20% en masse de phase dispersée à une vitesse uniforme et fixe et, avec agitation modérée, à un volume identique d'acétone, à 25°C, en 1 mn, et qu'on laisse ensuite au repos, à 25°C, en 1 mn, le produit de marquage restant stable en milieu aqueux, c'est-à-dire que la fluorescence du chélate ne disparait pas quand le produit de marquage est plongé, ou exposé d'une autre manière, à un milieu aqueux.

2. Produit de marquage conforme à la revendicaation 1, dans lequel le chélate de métal de terre rare fluorescent est un chélate d'europium ou de terbium.

3. Produit de marquage conforme à l'une des revendications 1 ou 2, dans lequel les particules de latex de polymère comprennent (1) un copolymère d'acrylate de n-butyle, de styrène et d'acide 2-acrylamido-2-méthylpropanesulfonique, (2) un copolymère d'acrylate de n-butyle et d'acide 2-acrylamido-2-méthylpropanesulfonique, (3) un terpolymère de styrène, de (2-chloroéthylsulfonyl-méthyl) styrène et d'acide 2-acrylamido-2-méthylpropanesulfonique, (4) un copolymère de styrène, d'acrylamide, de (2-chloroéthylsulfonylméthyl)-styrène et d'acide 2-acrylamido-2-méthylpropanesulfonique, (5) un copolymère d'acrylate de n-butyle, de styrène, de (m+p)-chloro-méthylstyrène et d'acide 2-acrylamido-2-méthylpropanesulfonique, (6) un copolymère d'acrylate de n-butyle, de styrène, de (m+p)chlorométhyl-styrène et de méthosulfate de 2-(méthacryloyloxy)éthyl-triméthylammonium, ou (7) un copolymère de styrène, d'acrylamide, et de (2-chloroéthylsulfonyl-méthyl)styrène.

4. Produit de marquage conforme à l'une quelconque des revendications 1 à 3, dans lequel le chélate de métal de terre rare fluorescent est formé à partir d'un métal de terre rare et d'un agent de chélation qui est une 1,3-diacétone, un phtalate, un naphtalate, une di-pyridine, une terpyridine, un p-benzoylbenzoate, un p-benzoylacétonate ou une phénanthraline.

5. Protéine marquée par fluorescence comprenant une protéine adsorbée ou liée par covalence à un produit de marquage fluorescent, stabilisé en milieu aqueux, conforme à l'une des revendications 1 à 4.

6. Protéine marquée, conforme à la revendication 5, dans laquelle la protéine est un anticorps, un antigène ou une enzyme.

7. Réactif immunologique marqué par fluorescence comprenant un antigène ou un anticorps adsorbé ou lié par covalence à un produit de marquage fluorescent, stabilisé en milieu aqueux, conforme à l'une des revendications 1 à 4.

8. Procédé pour préparer un produit de marquage fluorescent, stabilisé en milieu aqueux, conforme à l'une des revendications 1 à 4, qui comprend la formation d'une solution d'un chélate d'un métal de terre rare dans un solvant miscible à l'eau, puis l'addition d'un polymère pouvant être chargé, tel que défini à la revendication 1, le chélate de métal de terre rare fluorescent étant ainsi incorporé dans les particules hydrophobes du latex à une concentration pouvant atteindre 7,5% de la masse des particules.

9. Procédé pour préparer un réactif immunologique marqué, conforme à la revendication 7, selon lequel on mélange un produit de marquage fluorescent, stabilisé en milieu aqueux, conformé à l'une quelconque des revendications 1 à 4, à une solution aqueuse d'un antigène ou d'un anticorps.

10. Procédé pour réaliser une détermination immunologique où l'on utilise un réactif immunologique marqué, conforme à la revendication 7, et où, après mélange avec un échantillon d'essai, on détermine la fluorescence du liquide surnageant ou, le cas échéant, du précipité.

11. Procédé conforme à la revendication 10, où l'on mesure la fluorescence par une méthode de résolution temporelle.

12. Procédé pour réaliser une détermination immunologique où l'on utilise des réactifs immunologiques marqués par fluorescence, caractérisé en ce que le réactif immunologique marqué est un antigène ou un anticorps adsorbé ou lié par covalence à un produit de marquage fluorescent, sous forme de particules, stabilisé en milieu aqueux, conforme à la revendication 1.